(19) Europäisches Patentamt
European Patent Office
Office européen des brevets

(11) Veröffentlichungsnummer: **0 681 832 A2**

(12) # EUROPÄISCHE PATENTANMELDUNG

(21) Anmeldenummer: **95103283.8**

(51) Int. Cl.[6]: **A61K 7/50**

(22) Anmeldetag: **08.03.95**

(30) Priorität: **11.05.94 DE 4416566**

(43) Veröffentlichungstag der Anmeldung:
**15.11.95 Patentblatt 95/46**

(84) Benannte Vertragsstaaten:
**AT BE CH DE DK ES FR GB IT LI NL SE**

(71) Anmelder: **HÜLS AKTIENGESELLSCHAFT**
**D-45764 Marl (DE)**

(72) Erfinder: **Balzer, Dieter, Dr.**
**Talstrasse 21**
**D-45721 Haltern (DE)**

(54) **Wässrige viskoelastische Tensidlösungen zur Haar- und Hautreinigung.**

(57) Die Erfindung betrifft wäßrige, viskoelastische Tensidlösungen zur Reinigung von Haar und Haut im wesentlichen enthaltend

A: 4 bis 25 Gew.-% anionisches Tensid,
B: 0 bis 10 Gew.-% betainisches Tensid,
C: 0 bis 20 Gew.-% nichtionisches Tensid,
D: 0 bis 6 Gew.-% Elektrolyt,
E: 0 bis 5 Gew.-% wasserlösliches Polymer,
F: 0 bis 5 Gew.-% weitere Inhaltsstoffe,

dadurch gekennzeichnet, daß die Summe aus A, B und C mindestens 10 Gew.-% beträgt und die Summe aus C, D und E zwischen 2 und 20 Gew.-%, bezogen jeweils auf die wäßrige Lösung, liegt.

Der Schermodul $G_o$ der Lösung liegt bei Temperaturen zwischen 20 und 40 °C und pH 4 bis 8 zwischen 50 und 500 Pa. Die Bedingungen für die Identität von Speichermodul G' und Verlustmodul G'' liegen im Kreisfrequenzbereich zwischen 0,1 und 60 rad$\cdot s^{-1}$.

Durch Einhalten dieser Bedingungen erhalten die Flüssigkeiten für die vorgesehenen Anwendungszwecke optimales Fließverhalten.

EP 0 681 832 A2

Die Erfindung betrifft wäßrige Haar- und Hautreinigungsmittel vom Typ Shampoo, Duschgel, Badegel bzw. allgemein wäßrige kosmetische Reinigungsflüssigkeiten mit gewöhnlich hohem Schäumvermögen, für die aus Applikationsgründen eine erhöhte Viskosität notwendig bzw. erwünscht ist.

Solche Systeme basierten in der Vergangenheit vorwiegend auf anionischen Tensiden, wie insbesondere Fettalkoholsulfaten bzw. Fettalkoholethersulfaten ggf. in Kombination mit Fettsäureamiden, also Tensidkombinationen, die sich mit Elektrolyten leicht verdicken lassen. Alternativ hat man in neuerer Zeit aus Gründen höherer Hautfreundlichkeit Carbobetaine und Sulfosuccinate mit hinzugenommen. Als Verdikkungsmittel dieser Systeme eignen sich ebenfalls Fettalkoholoxethylate mit relativ niedrigem Ethoxylierungsgrad [vgl. A. Behler et al., SÖFW 116, 60 (1990)] bzw. Alkylpolyglycoside (EP 0 070 074, EP 0 384 983).

Weiterhin werden in manchen Fällen wasserlösliche Polymere als Verdicker den vorwiegend anionischen Tensiden zugesetzt.

Bei allen diesen Formulierungen werden in der Literatur rheologisch nur Daten zur Viskosität diskutiert, Grenzen für eine etwaig vorhandene Elastizität bleiben hingegen unerwähnt.

Viskoelastische Tensidlösungen (H. Hoffmann und H. Rehage in Surfactant Solutions, Surfactant Science Series Vol. 22, Ed. R. Zana, New York, 1987, S. 209 ff.), Systeme also, die sowohl ausgeprägte viskose wie elastische Eigenschaften haben, die bei der Zufuhr von mechanischer Energie also gleichzeitig mit der Umwandlung in Wärme und der Speicherung mechanischer Energie antworten, wurden vor allem bei quaternären Ammoniumverbindungen in Gegenwart stark bindender Gegenionen beobachtet. Typisch ist das Beispiel Cetylpyridiniumsalicylat. Aber auch andere kationische Tenside, wie etwa Hexadecyltrimethylammoniumbromid in Gegenwart extrem hoher Salzkonzentrationen, zeigen viskoelastisches Verhalten [A. Khatory et al. Langmuir 9, 1456 (1993)]. Anwendung finden solche Beobachtungen bei speziellen Reinigern, die möglichst unverdünnt durch unbewegtes Wasser zum Ort ihrer Wirkung zu transportieren sind, wie z. B. tensidarme, hochalkali- und elektrolythaltige Rohrreiniger (EP 0 317 066). In anderen Fällen wird das viskoelastische Verhalten von Reinigungslösungen durch zugesetzte stark vernetzte Polymere, die selbst deutlich elastisch wirken, erzwungen (EP 0 398 021, EP 0 584 877). Bei einfachen Tensidlösungen, wie insbesondere den anwendungstechnisch so bedeutsamen anionischen Tensiden, wurde viskoelastisches Verhalten bisher nicht beschrieben. Unter Tensidlösungen im Sinne dieser Erfindung werden Flüssigkeiten verstanden, die auch dispergierte Stoffe enthalten können.

Kürzlich haben wir nun beobachtet, daß bereits wäßrige Fettalkoholethersulfatlösungen bei für Personal-Care-Formulierungen üblichen Konzentrationen in Gegenwart üblicher Elektrolytmengen ausgeprägtes viskoelastisches Verhalten zeigen. Dieser Befund wurde ebenfalls bei Mischungen von Ethersulfaten mit anderen anionischen Tensiden oder mit Betainen oder mit Fettalkoholoxethylaten erhalten. Bei Kombinationen aus Fettalkoholethersulfaten mit Alkylpolyglykosiden wurde in manchen Fällen auch viskoelastisches Verhalten beobachtet, ohne daß hier Elektrolyte zugesetzt werden müssen. Durch Zusatz von typischen Polymeren oder anderen bei Personal-Care-Formulierungen üblichen Ingredienzien wird das viskoelastische Verhalten nicht grundsätzlich verändert, sondern meist nur modifiziert, wobei hier weniger an hochvernetzte Polymere mit extrem hohen Molmassen als an übliche Kettenmoleküle, insbesondere Naturprodukte oder modifizierte Naturprodukte, gedacht wird.

Die viskoelastischen Effekte beim Fließverhalten von wäßrigen Personal-Care-Lösungen haben erhebliche Bedeutung für deren Anwendung. Wesentlich sind hierbei vor allem zwei Prozesse, nämlich die Entnahme aus dem Vorratsgefäß und die Verteilung auf der Haut bzw. dem Haar. Während der erste Prozeß gewöhnlich bei relativ geringen Scherraten bis zu ca. 10 $s^{-1}$ abläuft, erfolgt die Verteilung auf der Haut bzw. dem Haar bei wesentlich höheren Krafteinwirkungen, gewöhnlich bei Scherraten oberhalb 100 $s^{-1}$. Die bisher ausschließlich auf Viskositätsdaten gestützte Diskussion forderte daher strukturviskoses Fließverhalten, wodurch ein langsamer Fluß aus dem Vorratsgefäß, eine einfache Dosierung verbunden mit dem Eindruck hoher Wirkstoffkonzentration, sowie eine einfache Verteilung auf der Haut gewährleistet sind. Da jedoch strukturviskose Tensidsysteme häufig viskoelastische Effekte zeigen und die elastischen Anteile mit steigenden Kräften meist stark zunehmen, ist die bisher geübte Diskussion unzureichend und muß auf der Basis der Viskoelastizitätsbetrachtung korrigiert werden. Demnach dürfen die elastischen Anteile nicht zu hoch werden, da sonst eine befriedigende Verteilung auf der Haut bzw. dem Haar erschwert wird. Außerdem führt hohe Elastizität beim Ausfluß der Flüssigkeit zu einem wenig ansprechenden Oszillieren des unförmig dicken Flüssigkeitsfadens im Gefäßhals; die Flüssigkeit strömt zurück, wenn die Gefäßneigung nur wenig zurückgenommen wird. Ausschließlich viskose Systeme hingegen neigen zur Bildung sehr langer, sich verjüngender Fäden, ebenfalls ein ästhetisch sehr unbefriedigendes Verhalten. Optimales Fließverhalten der Flüssigkeit wird man daher nur dann erwarten können, wenn Viskosität und Elastizität der Flüssigkeit aufeinander abgestimmt sind.

Die Aufgabe der vorliegenden Erfindung bestand daher darin, die Grenzen des viskoelastischen Verhaltens von Tensidlösungen aufzufinden, die ein optimales, d. h. vom Verbraucher erwünschtes, Fließverhalten gewährleisten.

Die Aufgabe wurde gelöst durch die erfindungsgemäßen Tensidlösungen und das Verfahren zur gezielten Herstellung dieser Tensidlösungen. Danach werden Lösungen aus 4 bis 25 % anionischem Tensid im wesentlichen mit 0 bis 10 % betainischem Tensid und/oder 0 bis 20 % nichtionischem Tensid und/oder 0 bis 6 % Elektrolyt und/oder 0 bis 5 % wasserlöslichen Polymeren und/oder 0 bis 5 % weiteren Inhaltsstoffen so zusammengesetzt, daß bei Temperaturen zwischen 20 und 40 °C und pH 4 bis 8 der Schermodul $G_o$ der Lösung zwischen 50 und 500 Pa sowie die Bedingungen für die Identität von Speichermodul G' und Verlustmodul G'' im Kreisfrequenzbereich zwischen 0,1 und 60 $rad \cdot s^{-1}$ liegen. Bevorzugt sind solche Lösungen, bei denen $G_o$ zwischen 70 und 450 Pa sowie die Bedingungen für die von G' und G'' im Kreisfrequenzbereich 0,3 bis 50 $rad \cdot s^{-1}$ liegen.

Die Erfindung betrifft daher wäßrige, viskoelastische Tensidlösungen zur Reinigung von Haar und Haut im wesentlichen enthaltend

A: 4 bis 25 Gew.-% anionisches Tensid,
B: 0 bis 10 Gew.-% betainisches Tensid,
C: 0 bis 20 Gew.-% nichtionisches Tensid,
D: 0 bis 6 Gew.-% Elektrolyt,
E: 0 bis 5 Gew.-% wasserlösliches Polymer,
F: 0 bis 5 Gew.-% weitere Inhaltsstoffe,

dadurch gekennzeichnet, daß die Summe aus A, B und C mindestens 10 Gew.-% beträgt und die Summe aus C, D und E zwischen 2 und 20 Gew.-%, bezogen jeweils auf die wäßrige Lösung, liegt und daß bei Temperaturen zwischen 20 und 40 °C und pH 4 bis 8 der Schermodul $G_o$ der Lösung zwischen 50 und 500 Pa sowie die Bedingungen für die Identität von Speichermodul G' und Verlustmodul G'' im Kreisfrequenzbereich zwischen 0,1 und 60 $rad \cdot s^{-1}$ liegen.

Bevorzugt sind die Mengen A: 5 bis 20 Gew.-%, B: 0 bis 8 Gew.-%, C: 0 bis 15 Gew.-%, D: 0 bis 5 Gew.-%, E: 0 bis 4 Gew.-%, wobei die Summe aus C, D und E bezogen auf die wäßrige Lösung 3 bis 15 Gew.-% betragen soll.

Weiterer Gegenstand der Erfindung ist ein Verfahren zur gezielten Herstellung wäßriger, viskoelastischer Tensidlösungen zur Reinigung von Haar und Haut im wesentlichen enthaltend

A: 4 bis 25 Gew.-% anionisches Tensid,
B: 0 bis 10 Gew.-% betainisches Tensid,
C: 0 bis 20 Gew.-% nichtionisches Tensid,
D: 0 bis 6 Gew.-% Elektrolyt,
E: 0 bis 5 Gew.-% wasserlösliches Polymer,
F: 0 bis 5 Gew.-% weitere Inhaltsstoffe,

dadurch gekennzeichnet, daß die Bestandteile aufgrund physikalischer Messungen so ausgewählt werden, daß die Summe aus A, B und C mindestens 10 Gew.-% beträgt und die Summe aus C, D und E zwischen 2 und 20 Gew.-%, bezogen jeweils auf die wäßrige Lösung, liegt, und daß bei Temperaturen zwischen 20 und 40 °C und pH 4 bis 8 der Schermodul $G_o$ der Lösung zwischen 50 und 500 Pa sowie die Bedingungen für die Identität von Speichermodul G' und Verlustmodul G'' im Kreisfrequenzbereich zwischen 0,1 und 60 $rad \cdot s^{-1}$ liegen.

Hierbei werden die anionischen Tenside ausgewählt aus den Gruppen Fettalkoholsulfate mit 10 bis 18 Kohlenstoffatomen in der Alkylgruppe, Fettalkoholethersulfate mit 1 bis 5 mol Ethylenoxid/mol und 10 bis 18 Kohlenstoffatomen in der Alkylgruppe, Monoalkyloxethylatsulfosuccinate mit 1 bis 7 mol Ethylenoxid/mol und 10 bis 18 Kohlenstoffatomen in der Alkylgruppe, Paraffinsulfonate auf der Basis $C_{12}$- bis $C_{20}$-Paraffin, Phosphorsäureester von $C_{10}$ - bis $C_{18}$-Fettalkoholen und auch Phosphorsäureester oxethylierter $C_{10}$- bis $C_{18}$-Fettalkohole mit 3 bis 20 mol Ethylenoxid/mol, carboxymethylierte $C_{10}$- bis $C_{18}$-Fettalkoholoxethylate mit 2 bis 20 mol Ethylenoxid/mol, $C_{10}$- bis $C_{18}$-Alkylisethionate und $C_{12}$- bis $C_{20}$-Olefinsulfonate sowie deren Gemischen. Besonders bevorzugt sind Fettalkoholethersulfate. Als Kationen werden Alkali-, Ammonium-, $C_1$- bis $C_3$-Alkylammonium- oder Magnesium-Ionen bevorzugt.

Unter betainischen Tensiden sind Alkylbetaine, Alkylamidobetaine sowie Imidazolinbetaine, alle mit mindestens einer langkettigen Alkylgruppe von 10 bis 18 Kohlenstoffatomen, sowie deren Mischungen zu verstehen (vgl. J. Falbe "Surfactants in Consumer Products", London 1987, S. 117 ff.). Als Beispiel für ein Alkylamidobetain sei das Kokoamidopropylbetain genannt.

Die nichtionischen Tenside werden ausgewählt aus den Gruppen Fettalkoholoxethylate, Alkylpolyglykoside, Fettsäure-N-alkyl-polyhydroxyamide, Aminoxide, Sorbitanester und deren Gemischen. Dabei entsprechen die Fettalkoholoxethylate der Formel I

I $R - O - (CH_2 - CH_2 - O)_m H$,

worin R einen gesättigten oder ungesättigten, verzweigten oder unverzweigten Alkylrest mit 10 bis 18 Kohlenstoffatomen und m eine Zahl zwischen 1 und 8 bedeuten. Bevorzugt werden für m Zahlen zwischen 2 und 6. Die Alkylpolyglykoside entsprechend der Formel II

II $R' - O - Z_n$,

in der R' gesättigte oder ungesättigte, verzweigte oder unverzweigte Alkylreste mit 10 bis 16 Kohlenstoffatomen und $Z_n$ ein Oligoglycosylradikal bestehend aus 1 bis 3 Hexose- oder Pentoseeinheiten bzw. Mischungen davon bedeuten. Bevorzugt sind Alkylglucoside mit Glucosidierungsgraden zwischen 1,1 und 2. Alkylpolyglucoside sind sehr umweltfreundliche neue Tenside, die inzwischen aber gut beschrieben sind [vgl. EP 0 070 074, EP 0 384 983; D. Balzer, Tenside Surf. Det. 28, 419 (1991) oder D. Balzer, Langmuir 9, 3375 (1993)].

N-Alkylpolyhydroxyfettsäureamide entsprechen der Formel III

III 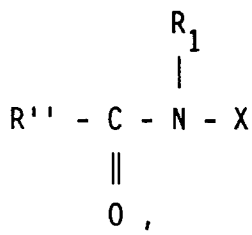

in der R'' einen verzweigten oder unverzweigten Alkylrest mit 8 bis 18 Kohlenstoffatomen, $R_1$ Methyl-, Ethyl-, n- oder i-Propyl und X ein Polyhydroxyhydrocarbyl mit mindestens 3 Hydroxygruppen oder Gemische davon bedeuten. Besonders bevorzugte Fettsäure-N-Alkylpolyhydroxyamide sind Fettsäure-N-Methylglucamide der Formel IIIa

IIIa 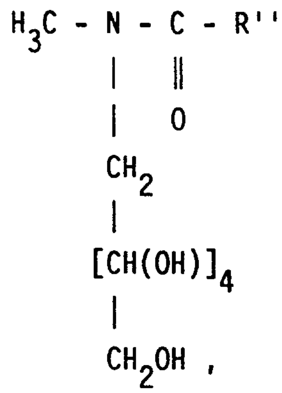

in der R'' einen verzweigten oder unverzweigten Alkylrest mit 10 bis 16 Kohlenstoffatomen bedeutet.

Die erfindungsgemäß einzusetzenden Verbindungen können bekanntermaßen durch Umsetzung von Fettsäuren bzw. Fettsäureestern mit ggf. N-substituiertem Polyhydroxyalkylamin in der Schmelze, ggf. in Gegenwart alkalischer Katalysatoren hergestellt werden (vgl. EP 0 285 768).

Aminoxide entsprechen der Formel IV

IV

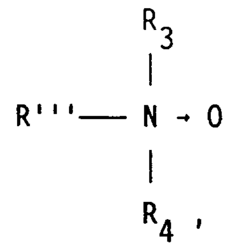

worin R''' einen gesättigten oder ungesättigten, verzweigten oder unverzweigten Alkylrest mit 10 bis 18 Kohlenstoffatomen und $R_3$ und $R_4$ kurzkettige $C_1$- bis $C_3$-Alkylreste bedeuten.

Bevorzugt werden $C_{12}$- bis $C_{18}$-Alkyldimethylaminoxide, die sich z. B. durch $H_2O_2$-Oxydation der diesbezüglichen tertiären Amine herstellen lassen (vgl. DE 30 14 510).

Sorbitanester werden u. a. von Atlas Powder Co. unter der Bezeichnung SPAN[R] bzw. TWEEN[R] vertrieben.

Als zuzusetzende Elektrolyte kommen alle Salze in Frage, die sowohl toxikologisch unbedenklich als auch mit den anderen Bestandteilen der Zubereitung verträglich sind. Bevorzugt sind dies NaCl und $NH_4Cl$, aber auch $MgCl_2$, $Na_2SO_4$ und auch Alkalisalze von Carbonsäuren wie Natriumcitrat.

Wasserlösliche Polymere dienen bei den erfindungsgemäßen Tensidlösungen einer ggf. zweckmäßigen gewöhnlich geringfügigen Modifizierung des rheologischen Profils, das im wesentlichen von der Kombination Tenside/Elektrolyt vorgegeben wird. Gemeint sind hier natürliche Polysaccharide bzw. ihre Derivate wie Xanthan, Guarderivate, Natriumalginat, Cellulosederivate wie Hydroxypropyl- oder Hydroxyethylcellulose, aber auch Polyethylenoxide und Polyacrylate. Die Molmassen liegen zwischen 200 000 und 3 000 000 g/mol. Bei Derivaten von Polyethylenoxid bzw. oxethylierten mehrwertigen Alkoholen entsprechend EP 0 511 466 liegen auch niedrigere Molmassen vor. Diese Stoffe oder ihre Gemische werden in Konzentrationen bis zu 5 %, vorzugsweise bis zu 4 %, der erfindungsgemäßen Tensidlösung zugesetzt.

Weitere Inhaltsstoffe sind Konditioniermittel (insbesondere kationische Polymere wie z. B. kationische Guartypen oder Hydroxethylcellulosen bzw. auch andere kationische Polymere entsprechend EP 0 337 354), quaternäre Tenside z. B. vom Typ langkettiger Alkyltrimethylamnoniumchloride, Silikonöle vom Typ Dimethylpolysiloxan, rückfettende Substanzen wie Glyceride vom MIGLYOL[R]-, SOFTIGEN[R]- oder SOFTISAN[R]-Typ, besonders hautverträgliche Stoffe wie Eiweißfettsäurekondensate, herstellbar z. B. durch Umsetzung von Pflanzen- oder Tierproteinhydrolysaten und Fettsäurechloriden. Weitere Inhaltsstoffe sind auch spezielle medizinisch wirksame Verbindungen, Puffersubstanzen, Konservierungsmittel und Farbstoffe. Die erfindungsgemäßen Reinigungsflüssigkeiten werden auf einen hautfreundlichen, gewöhnlich schwach-sauren pH-Wert eingestellt.

Die erfindungsgemäß charakteristischen Größen für das jeweilige System $G_o$ und die Strukturrelaxationszeit $\tau$ bzw. ihr reziproker Wert resultieren aus Gleichung (5) bzw. (7). Da G' auch bei Nicht-Maxwell'schen Verhalten bei höheren Frequenzen einem Sättigungswert $G_o$ zustrebt, gilt Gleichung (5) auch für solche Systeme.

Die erfindungsgemäßen Komponenten der wäßrigen Tensidlösung werden unter Rühren - ggf. nach vorheriger Lösung in Wasser - zusammengegeben und nach einer Equilibrierungszeit von wenigstens 24 h und sorgfältig geprüfter Blasenfreiheit der rheologischen Untersuchung unterworfen. Hierzu dienen Oszillationsviskosimeter (u. a. Haake CV 20), die die gleichzeitige Ermittlung von elastischem und viskosem Anteil gestatten. Dies setzt voraus, daß die Messung im linear-viskoelastischen Bereich durchgeführt wird, der sich mittels Deformationstest ermitteln läßt. Mit der so festgestellten maximalen Amplitude wird die Oszillation der Reinigungslösung bei konstanter Temperatur über den gesamten Frequenzbereich durchgeführt. Registriert werden der Speichermodul G' als elastischer und der Verlustmodul G'' als viskoser Anteil der dem System zugeführten Energie. Es wurde festgestellt, daß sich die erfindungsgemäßen wäßrigen Tensidlösungen in den meisten Fällen wie Maxwell'sche Flüssigkeiten (H. Hoffmann und H. Rehage, loc. cit.) verhalten (vgl. Abb. 1). Danach gilt für

$$(1)\ G' = \frac{G_o \omega^2 \tau^2}{1 + \omega^2 \tau^2}$$

und für

$$(2)\ G'' = \frac{G_o\ \omega \cdot \tau}{1 + \omega^2\ \tau^2},$$

worin $\omega$ die Kreisfrequenz ($rad \cdot s^{-1}$), $\tau$ (s) die Strukturrelaxationszeit und $G_o$ (Pa) den Schermodul bedeuten.

Interessante Grenzfälle sind zu berücksichtigen:

Für niedrige Frequenzen $\omega \cdot \tau \ll 1$

$$G' \approx \omega^2 \quad (3)$$

$$G'' \approx \omega \quad (4)$$

und für hohe Frequenzen $\omega \cdot \tau \gg 1$

$$G' \approx G_o. \quad (5)$$

Für den Fall G' = G'' folgt

$$G_o = 2\ G' \text{ und} \quad (6)$$

$$\tau = \frac{1}{\omega}, \quad (7)$$

wobei $\tau$ die Struktur-Relaxationszeit der Flüssigkeit ist.

Beispiele

Die nachstehenden Beispiele sollen das erfindungsgemäße Verfahren erläutern.

Die Lösungen wurden auf pH 6 eingestellt sowie bei 25 °C rheologisch vermessen. Die Tabelle enthält neben überwiegend erfindungsgemäßen Beispielen auch einige Vergleichsbeispiele, denen Reinigungsflüssigkeiten mit unzureichenden rheologischen Eigenschaften entsprechen, weil sie entweder zu geringe elastische Anteile aufweisen (Beispiel 2 und 5) bzw. ihr $\omega$-Wert zu hoch (Beispiel 20) oder zu niedrig (Beispiel 18) liegt.

Die in der folgenden Tabelle verwendeten Abkürzungen haben folgende Bedeutung:

| | |
|---|---|
| FAES: | $C_{12}C_{14}$-Fettalkoholethersulfat-Natriumsalz mit 2 mol Ethylenoxid/mol |
| FAECM: | carboxymethyliertes $C_{12}C_{14}$-Fettalkoholoxethylat-Natriumsalz mit 4,5 mol Ethylenoxid/mol |
| FASS: | $C_{12}C_{14}$-Fettalkoholoxethylatsulfosuccinat-Natriumsalz mit 3 mol Ethylenoxid/mol |
| Betain: | $C_{12}C_{14}$-Fettsäureamidopropylbetain |
| FAO: | $C_{12}C_{14}$-Fettalkoholoxethylat mit 3 mol Ethylenoxid/mol |
| APG: | $C_{12}C_{14}$-Alkylpolyglucosid mit einem Glucosidierungsgrad von 1,2 |
| FAGA: | $C_{12}$-Fettsäure-N-methylglucamid |
| PEG: | POLYOX$^R$ Resin WSR N-60 K, Union Carbide |
| Guarderivat: | JAGUAR$^R$ HP 60, Meyhall |
| Xanthan: | KELTROL$^R$ T, Kelco |
| Silikonöl: | AK 100, Wacker |
| JR 400: | UCARE$^R$ JR 400 von Amercol |
| Eiweißfettsäurekondensat: | LAMEPON$^R$ S, Henkel |
| SOFTIGEN$^R$ 767: | Oxethyliertes Capryl-Caprinssäurepartialglycerid, Hüls AG |

Tabelle (1. Teil)

| Beispiel / Komponenten | 1 | 2 (V) | 3 | 4 | 5 (V) | 6 |
|---|---|---|---|---|---|---|
| FAES | 15 | 15 | 14 | 17 | 23 | 6 |
| FAECM | - | - | - | - | - | - |
| FASS | - | - | - | - | - | - |
| BETAIN | - | - | - | - | - | - |
| FAO | | - | 3,5 | 4 | 6 | - |
| APG | | | | | | 19 |
| FAGA | - | - | - | - | - | - |
| NaCl | 5 | 2 | 2 | 2 | 3 | - |
| PEG | - | - | - | - | - | - |
| Guar | - | - | - | - | - | - |
| Xanthan | - | - | - | - | - | - |
| Silikonöl | - | - | - | - | - | - |
| JR 400 | - | - | - | - | - | - |
| Eiweißfettsäurekondensat | - | - | - | - | - | - |
| SOFTIGEN R 767 | - | - | - | - | - | - |
| $G_o$ (max) (Pa) | 280 | ≈10 | 280 | 400 | - | 400 |
| $\omega_{G' = G''}$ ($rad \cdot s^{-1}$) | 7 | 142 | 14 | 18 | 130 | 23 |

Tabelle (2. Teil)

| Beispiel / Komponenten | 7 | 8 | 9 | 10 | 11 | 12 |
|---|---|---|---|---|---|---|
| FAES | 4 | 7,5 | 8,5 | 10 | 7,5 | 10 |
| FAECM | - | - | 3,0 | - | - | - |
| FASS | - | - | - | - | 7,5 | - |
| BETAIN | - | - | - | 2 | - | 2 |
| FAO | | - | - | - | - | - |
| APG | 13 | 7,5 | 8,5 | 5 | - | - |
| FAGA | - | - | - | - | 2 | 5 |
| NaCl | - | 2 | 2 | 1 | 4 | - |
| PEG | - | - | - | - | - | - |
| Guar | - | - | - | - | - | - |
| Xanthan | - | - | - | - | - | - |
| Silikonöl | - | - | - | - | - | - |
| JR 400 | - | - | - | - | - | - |
| Eiweißfettsäurekondensat | - | - | - | - | - | - |
| SOFTIGEN$^{R}$ 767 | - | - | - | - | - | - |
| $G_0$ (max) (Pa) | 100 | 180 | 200 | 180 | 110 | 160 |
| $\omega_{G' = G''}$ (rad·s$^{-1}$) | 15 | 20 | 22 | 24 | 43 | 22 |

Tabelle (3. Teil)

| Beispiel / Komponenten | 13 | 14 | 15 | 16 | 17 | 18(V) |
|---|---|---|---|---|---|---|
| FAES | 12 | 5 | 7,5 | 7,5 | 7,5 | 15 |
| FAECM | - | - | - | - | - | - |
| FASS | - | - | - | - | - | - |
| BETAIN | 5 | 2 | - | - | - | - |
| FAO | - | - | - | - | - | - |
| APG | - | - | 7,5 | 7,5 | 7,5 | - |
| FAGA | - | 10 | - | - | - | - |
| NaCl | 3 | - | 1 | 2 | 2 | 0,5 |
| PEG | - | - | - | - | - | - |
| Guar | - | - | - | - | - | 2,5 |
| Xanthan | - | - | 1 | - | - | - |
| Silikonöl | - | - | - | - | - | - |
| JR 400 | - | - | - | 2 | 2 | - |
| Eiweißfettsäurekondensat | - | - | - | - | 1 | - |
| SOFTIGEN R 767 | - | - | - | - | - | - |
| $G_0$ (max) (Pa) | 200 | 400 | 80 | 300 | 320 | 230 |
| $\omega_{G' = G''}$ ($rad \cdot s^{-1}$) | 5 | 25 | 50 | 6 | 34 | 0,02 |

Tabelle (4. Teil)

| Beispiel / Komponenten | 19 | 20 (V) | 21 | 22 | 23 |
|---|---|---|---|---|---|
| FAES | 15 | 15 | 10 | 10 | 8 |
| FAECM | - | - | 7 | 7 | - |
| FASS | - | - | - | - | - |
| BETAIN | - | - | 2 | 2 | 4 |
| FAO | - | - | - | - | - |
| APG | - | - | - | - | 4 |
| FAGA | - | - | - | - | 1 |
| NaCl | 0,5 | 0,5 | 2,8 | 2,8 | 2 |
| PEG | - | 1,5 | - | - | - |
| Guar | 1,5 | - | - | - | - |
| Xanthan | - | - | - | - | - |
| Silikonöl | - | - | - | - | - |
| JR 400 | - | - | - | - | 1 |
| Eiweißfettsäurekondensat | - | - | 2 | 2 | 2 |
| SOFTIGEN R 767 | - | - | - | - | 1 |
| $G_0$ (max) (Pa) | 140 | 100 | 175 | 200 | 200 |
| $\omega_{G' = G''}$ $(rad \cdot s^{-1})$ | 1 | 60 | 50 | 7 | 41 |

## Patentansprüche

1. Wäßrige, viskoelastische Tensidlösungen zur Reinigung von Haar und Haut im wesentlichen enthaltend
   A: 4 bis 25 Gew.-% anionisches Tensid,
   B: 0 bis 10 Gew.-% betainisches Tensid,
   C: 0 bis 20 Gew.-% nichtionisches Tensid,
   D: 0 bis 6 Gew.-% Elektrolyt,
   E: 0 bis 5 Gew.-% wasserlösliches Polymer,
   F: 0 bis 5 Gew.-% weitere Inhaltsstoffe,
   dadurch gekennzeichnet, daß die Summe aus A, B und C mindestens 10 Gew.-% beträgt und die Summe aus C, D und E zwischen 2 und 20 Gew.-%, bezogen jeweils auf die wäßrige Lösung, liegt,

und daß bei Temperaturen zwischen 20 und 40 °C und pH 4 bis 8 der Schermodul $G_o$ der Lösung zwischen 50 und 500 Pa sowie die Bedingungen für die Identität von Speichermodul G' und Verlustmodul G" im Kreisfrequenzbereich zwischen 0,1 und 60 rad•$s^{-1}$ liegen.

**2.** Wäßrige, viskoelastische Tensidlösung nach Anspruch 1,
dadurch gekennzeichnet,
daß bei Temperaturen zwischen 20 und 40 °C und pH 4 bis 8 der Schermodul $G_o$ der Lösung zwischen 70 und 450 Pa sowie die Bedingungen für die Identität von G' und G" im Kreisfrequenzbereich zwischen 0,3 und 50 rad•$s^{-1}$ liegen.

**3.** Wäßrige, viskoelastische Tensidlösung nach den Ansprüchen 1 und 2,
dadurch gekennzeichnet,
daß die anionischen Tenside aus den Gruppen Fettalkoholsulfate mit 10 bis 18 Kohlenstoffatomen in der Alkylgruppe, Fettalkoholethersulfate mit 1 bis 5 mol Ethylenoxid/mol und 10 bis 18 Kohlenstoffatomen in der Alkylgruppe, carboxymethylierte Fettalkoholoxethylate mit 2 bis 20 mol Ethylenoxid/mol, Monoalkyloxethylatsulfosuccinate mit 1 bis 7 mol Ethylenoxid/mol und 10 bis 18 Kohlenstoffatomen in der Alkylgruppe, Paraffinsulfonate mit 12 bis 20 Kohlenstoffatomen in der Alkylgruppe, Phosphorsäureester von $C_{10}$- bis $C_{18}$-Fettalkoholen und Phosphorsäureester oxethylierter $C_{10}$- bis $C_{18}$-Fettalkohole, $C_{10}$- bis $C_{18}$-Alkylisethionate sowie Olefinsulfonate mit 10 bis 20 Kohlenstoffatomen in den Alkyl- bzw. Alkylengruppen und Alkali-, Ammonium-, $C_1$-bis $C_3$-Alkylammonium bzw. Mg als Gegenionen bzw. ihren Gemischen ausgewählt werden.

**4.** Wäßrige, viskoelastische Tensidlösung nach den Ansprüchen 1 bis 3,
dadurch gekennzeichnet,
daß als betainische Tenside Alkylamidobetaine, Alkylbetaine sowie Imidazolinbetaine jeweils mit 10 bis 18 Kohlenstoffatomen in der Alkylgruppe bzw. deren Gemische eingesetzt werden.

**5.** Wäßrige, viskoelastische Tensidlösung nach den Ansprüchen 1 bis 3,
dadurch gekennzeichnet,
daß als nichtionische Tenside Fettalkoholoxethylate, Alkylpolyglucoside, Fettsäure-N-alkylpolyhydroxyamide, Aminoxide, Sorbitanester und deren Gemische verwendet werden.

**6.** Wäßrige, viskoelastische Tensidlösung nach den Ansprüchen 1 bis 3 und 5,
dadurch gekennzeichnet,
daß die Fettalkoholoxethylate durch die Formel I

I $R - O - (CH_2 - CH_2 - O)_m H$,

in der R gesättigte oder ungesättigte, verzweigte oder unverzweigte Alkylreste mit 10 bis 18 Kohlenstoffatomen und m eine Zahl zwischen 1 und 8 ist, beschrieben werden.

**7.** Wäßrige, viskoelastische Tensidlösung nach den Ansprüchen 1 bis 3 und 5,
dadurch gekennzeichnet,
daß die Alkylpolyglycoside durch die Formel II

II $R^1 - O - Z_n$,

in der R' gesättigte oder ungesättigte, verzweigte oder unverzweigte Alkylreste mit 10 bis 16 Kohlenstoffatomen und $Z_n$ ein Oligoglycosylradikal bestehend aus 1 bis 3 Hexose- oder Pentoseeinheiten bzw. Mischungen davon bedeuten, beschrieben werden.

**8.** Wäßrige, viskoelastische Tensidlösung nach den Ansprüchen 1 bis 3 und 5,
dadurch gekennzeichnet,
daß die N-Alkylpolyhydroxyfettsäureamide der Formel III

III 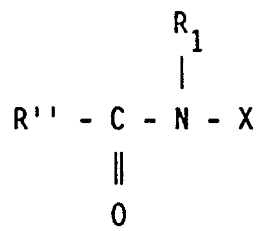

entsprechen,
in der R'' einen verzweigten oder unverzweigten Alkylrest mit 8 bis 18 Kohlenstoffatomen, $R_1$ Methyl-, Ethyl-, n- oder i-Propyl und X ein Polyhydroxyhydrocarbyl mit mindestens 3 Hydroxygruppen oder Gemische davon bedeuten.

**9.** Wäßrige, viskoelastische Tensidlösung nach Anspruch 8,
dadurch gekennzeichnet,
daß die Fettsäure-N-Alkylpolyhydroxyamide Fettsäure-N-Methylglucamide der Formel IIIa

IIIa 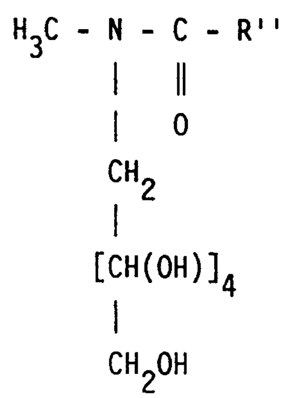

entsprechen, in der R'' einen verzweigten oder unverzweigten Alkylrest mit 10 bis 16 Kohlenstoffatomen bedeutet.

**10.** Wäßrige, viskoelastische Tensidlösung nach den Ansprüchen 1 bis 3 und 5,
dadurch gekennzeichnet,
daß die Alkylaminoxide der Formel IV

IV 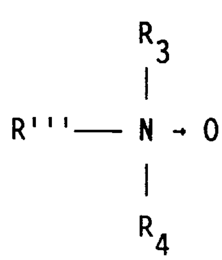

entsprechen, in der R''' einen gesättigten oder ungesättigten, verzweigten oder unverzweigten Alkylrest mit 10 bis 18 Kohlenstoffatomen und $R_3$ bzw. $R_4$ Methyl-, Ethyl-, n- oder i-Propyl bedeuten.

**11.** Wäßrige, viskoelastische Tensidlösung nach den Ansprüchen 1 und 2,
dadurch gekennzeichnet,
daß als Elektrolyt Alkali-, Ammonium-, Magnesiumchlorid, Alkalisulfat oder carbonsaure Alkalisalze

sowie deren Mischungen verwendet werden.

**12.** Wäßrige, viskoelastische Tensidlösung nach den Ansprüchen 1 und 2,
dadurch gekennzeichnet,
daß als wasserlösliche Polymere Polysaccharide vom Xanthan bzw. Guartyp, deren Derivate, Cellulosederivate, Polyethylenoxide sowie Polyacrylate eingesetzt werden.

**13.** Wäßrige, viskoelastische Tensidlösung nach Anspruch 12,
dadurch gekennzeichnet,
daß die Polymere Molmassen zwischen 200 000 und 3 000 000 g/mol besitzen.

**14.** Wäßrige, viskoelastische Tensidlösung nach den Ansprüchen 1 bis 13,
dadurch gekennzeichnet,
daß als weitere Inhaltsstoffe Rückfetter, Konditioniermittel, medizinische Wirkstoffe, Puffersubstanzen, Farbstoffe, Parfums und Konservierungsstoffe eingesetzt werden.

**15.** Verfahren zur gezielten Herstellung wäßriger, viskoelastischer Tensidlösungen zur Reinigung von Haar und Haut im wesentlichen enthaltend
A: 4 bis 25 Gew.-% anionisches Tensid,
B: 0 bis 10 Gew.-% betainisches Tensid,
C: 0 bis 20 Gew.-% nichtionisches Tensid,
D: 0 bis 6 Gew.-% Elektrolyt,
E: 0 bis 5 Gew.-% wasserlösliches Polymer,
F: 0 bis 5 Gew.-% weitere Inhaltsstoffe,
dadurch gekennzeichnet, daß die Bestandteile aufgrund physikalischer Messungen so ausgewählt werden, daß die Summe aus A, B und C mindestens 10 Gew.-% beträgt und die Summe aus C, D und E zwischen 2 und 20 Gew.-%, bezogen jeweils auf die wäßrige Lösung, liegt, und daß bei Temperaturen zwischen 20 und 40 °C und pH 4 bis 8 der Schermodul $G_o$ der Lösung zwischen 50 und 500 Pa sowie die Bedingungen für die Identität von Speichermodul G' und Verlustmodul G'' im Kreisfrequenzbereich zwischen 0,1 und 60 $rad \cdot s^{-1}$ liegen.

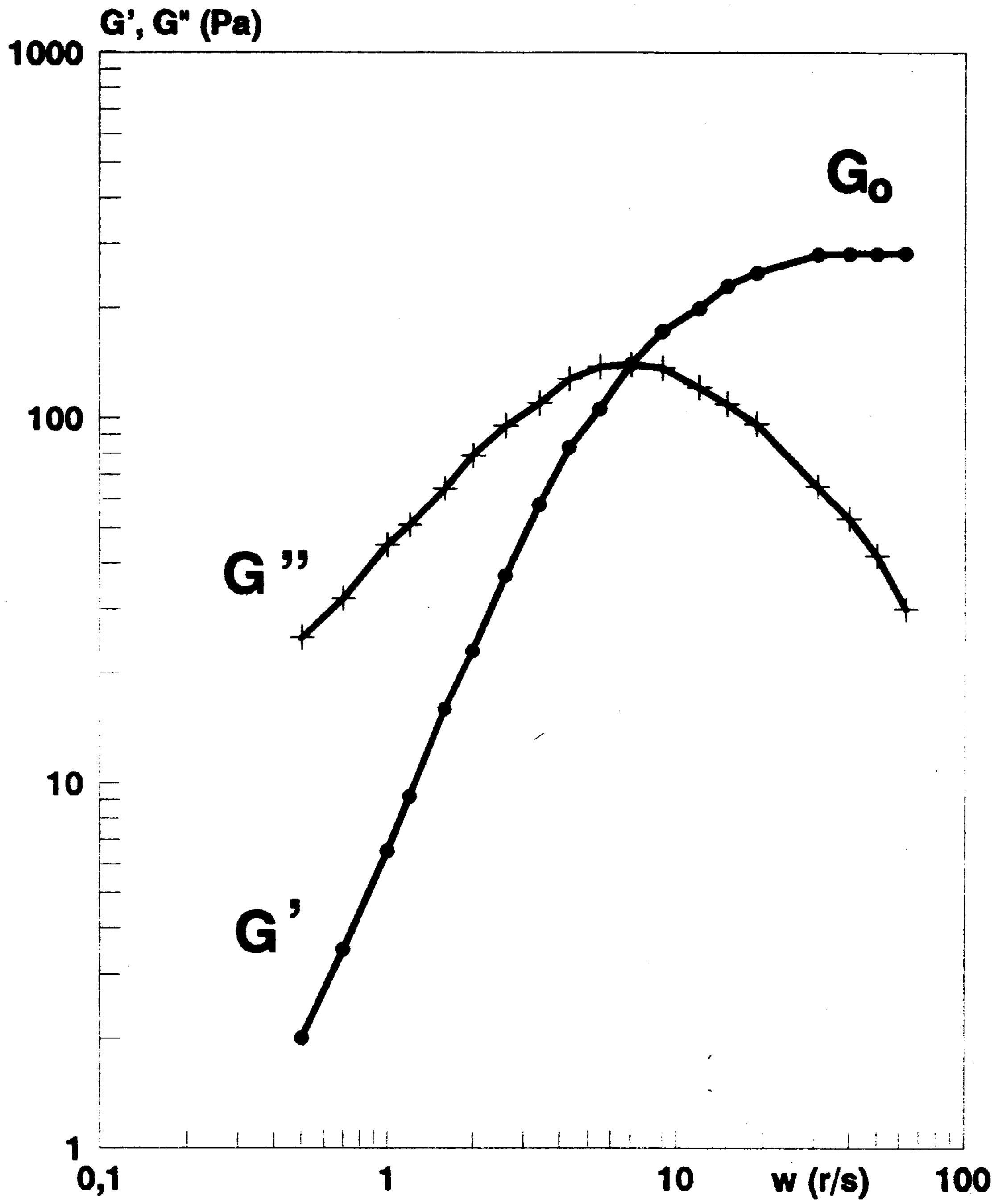


**G' und G" in Abhängigkeit der Kreisfrequenz bei 15% FAES/5% NaCl**
**Amplitude 4 Grad**